# EUROPEAN PATENT APPLICATION

(11) **EP 2 597 467 A1**
(43) Date of publication of application: **29.05.2013**
(21) Application number: 12173646.6
(22) Date of filing: 26.06.2012
(51) Int. Cl.: G01N 33/68

(54) **Means and methods for proSP-B based diagnosis of pulmonary congestion in ACS patients**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to the field of diagnostics. In particular, it relates to a method for diagnosing whether a subject exhibiting a symptom of an acute cardiovascular event suffers from a pulmonary complication, or not, said method comprising determining the amount of a surfactant protein-B peptide (SP-B peptide) in a sample of said subject, and comparing said amount to a reference amount whereby it is diagnosed whether the said subject suffers from a pulmonary complication, or not. Further contemplated is a method for diagnosing whether a subject exhibiting a symptom of an acute cardiovascular event is at risk of developing a pulmonary complication, or not. Moreover, the invention relates to a device or kit for carrying out the method of the invention.

## Description

The present invention relates to the field of diagnostics. In particular, it relates to a method for diagnosing whether a subject exhibiting a symptom of an acute cardiovascular event suffers from a pulmonary complication, or not, said method comprising determining the amount of a surfactant protein-B peptide (SP-B peptide) in a sample of said subject, and comparing said amount to a reference amount whereby it is diagnosed whether the said subject suffers from a pulmonary complication, or not. Further contemplated is a method for diagnosing whether a subject exhibiting a symptom of an acute cardiovascular event is at risk of developing a pulmonary complication, or not. Moreover, the invention relates to a device or kit for carrying out the method of the invention.

Chest pain and acute coronary syndrome, as characterised by prolonged chest pain (more than 20 minutes) at rest or after exercise, are frequent events specifically in the elderly. Individuals experiencing such symptoms frequently suffer from coronary artery disease and harbour diseases such as diabetes mellitus, systemic hypertension, obesity and others and may have risk factors such as smoking. If chest pain can be linked to cardiac ischemia affected sections of the heart may become dysfunctional (stunning effects) and this in turn may worsen cardiac function and results in cardiac complications such as cardiac shock which is easily recognised by pulselessness. Another complication in this context is pulmonary congestion which may occur as a consequence of the event or as a result of treatment. Therefore it is important to identify patients at risk of pulmonary congestion or related pulmonary complications in case they develop chest pain or acute coronary syndrome. Alveolar-capillary dysfunction resulting in pulmonary complications may also occur in in patients suffering from chronic heart failure (Guazzi 2005, Therapy 2(4): 641-648).

Surfactant Proteins and, in particular, SP-B have been reported to be biomarkers for lung diseases or disorders. EP 1 845 380 A and EP 1 882 945 A disclose that mature SP-B in combination with other biomarkers can be used to differentiate between several causes of shortness of breath. WO1999/133337 discloses that SP-B can be used for the diagnosis of the extent of lung damage. WO2004/077056 discloses that SP-B levels can be used to assess heart failure. Guazzi et al. (Guazzi loc cit) discloses that elevated SP-B levels can, in general, be associated with alveolar-capillary membrane damage in patients with pulmonary cardiogenic edema.

The technical problem underlying the present invention can be seen as the provision of means and methods for reliably and efficiently diagnosing pulmonary complications and, in particular pulmonary congestion, in subjects suffering from or suspected to suffer from an acute cardiovascular event, such as acute coronary syndrome (ACS). The technical problem is solved by the embodiments characterized in the claims and herein below.

Thus, the present invention relates to a method for diagnosing whether a subject exhibiting a symptom of an acute cardiovascular event suffers from a pulmonary complication, or not, said method comprising:
a) determining the amount of a surfactant protein-B peptide (SP-B peptide) in a sample of said subject; and
b) comparing said amount to a reference amount whereby it is diagnosed whether the said subject suffers from a pulmonary complication, or not.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein means assessing whether a subject as referred to herein suffers from the pulmonary complication, or not. As will be understood by those skilled in the art, such an assessment is usually not intended to be correct for 100% of the subjects to be diagnosed. The term, however, requires that assessment of the presence or absence of a pulmonary complication is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "a symptom of an acute cardiovascular event" as used herein refers to symptoms accompanying an acute coronary syndrome (ACS) or myocardial infarction. Preferably, the symptom referred to in accordance with the present invention is chest pain. More preferably, the chest pain occurs for a prolonged time (e.g., for at least 20 min) at rest or after exercise. Another symptom referred to in accordance with the present invention which may occur independently or together with the chest pain is cardiac ischemia. Preferably, cardiac ischemia can be determined by elevated amounts of sFlt-1 in the blood (see, e.g., WO2010/144553 or W02011/064358). Moreover, further symptoms include an elevation of parameters indicating impaired cardiac function, such as elevated amounts of natriuretic peptides determined in the blood, or cardiac damage, such as the cardiac troponins. Preferably, the subject which exhibits a symptom of an acute cardiovascular event as referred to herein suffers from an ACS, ST-elevation myocardial infarction (STEMI), heart failure and, preferably decompensated heart failure, diabetes mellitus, systemic hypertension, obesity or cardiac ischemic conditions. Moreover, subjects may also include those with risk factors for the said diseases such as previous smokers (i.e. smokers which are non-smokers, preferably, for at least 2weeks). Preferably, the subject referred to herein exhibits at least chest pain or suffers from STEMI. Moreover, the subject may suffer from heart failure as set forth in the accompanying Examples, below.

The term "pulmonary complication" refers to a damage or an impairment of the alveo-capillary membrane in the lung which results in an impaired pulmonary hemodynamic. Preferably, the pulmonary complication is pulmonary congestion and/or pulmonary edema. Subjects which suffer from pulmonary edema exhibit as further lung specific symptoms rales and can be confirmed to suffer from pulmonary edema by chest x-raying. Pulmonary congestion may be clinically apparent or subclinical. The clinically apparent form of pulmonary congestion can be confirmed by rales and dyspnea.

The term "subject" as used herein relates to animals, preferably mammals, and, more preferably, humans. The subject according to the present invention shall exhibit a symptom of an acute cardiovascular event as described elsewhere herein and, preferably, suffers from ACS or STEMI. Preferably, said subject does, however, not exhibit one or more other diseases, disorders or life style behaviours selected from the group consisting of: lung diseases, preferably asthma bronchiale or chronic obstructive pulmonary disease (COPD), pneumonia, current intoxications by substances, preferably, by drugs, such as bleomycin or methothrexat, lung cancer, acute systemic infections, current smokers, and kidney disease.

It is known that several diseases, disorders or life style behaviours, such as mentioned above, can also cause an elevation of SP-B peptides in the blood. Accordingly, the method of the present invention shall, preferably, not be applied to those subjects. If the clinical history of a subject to be investigated by the method of the present invention with respect to the aforementioned diseases, disorders or life style behaviours is unknown, the subject may, in a preferred embodiment of the method of the present invention, be tested for the presence of the said disease, disorders and/or life style behaviours as set forth elsewhere herein.

The term "sample" refers to a sample of a body fluid. Samples of body fluids can be obtained by well known techniques and include, preferably, samples of blood, plasma, serum, or urine, more preferably, samples of blood, plasma or serum.

The term "SP-B peptide" relates to peptides which are derived from the precursor polypeptide of the Surfactant Protein B (SP-B). SP-B is synthesised as a precursor, i.e. proSP-B, and the mature SP-B is obtained by proteolytic cleavage. The mature SP-B is a 79-amino acid hydrophobic peptide that facilitates the stability and rapid spreading of surfactant phospholipids during respiratory cycles. It maintains the molecular continuity of the monolayer of the lipid and peptide at the air-water interface during breathing and facilitates the incorporation of lipids from the lung aqueous subphase into the lipid monolayer at the alveolar air-water interface. Since mature SP-B is comprised in the proSP-B in its N-terminal portion, proSP-B is, preferably, determined by detecting the presence or absence of the C-terminal portion of the proSP-B polypeptide, i.e. C-terminal proSP-B. A preferred "SP-B" in the sense of the present invention is proSP-B, preferably C-terminal proSP-B. C-terminal proSP-B in the sense of the present invention relates to proSP-B and all cleavage products or fragments thereof comprising the C-terminal sequence of full length proSP-B. Accordingly, C-terminal proSP-B includes but is not limited to the following: (i) full length proSP-B, i.e. proSP-B comprising the N-terminal propeptide including the amino acid sequence of mature SP-B and the C-terminal proSP-B, (ii) the mid-molecular portion and the C-terminal fragment, i.e. the amino acids from position 201 to 381of proSP-B and (iii) the C-terminal proSP-B fragment, i.e. the amino acids from position 280 to 381 of proSP-B. Mature SP-B may also preferably be determined. Amino acid sequences comprising amino acids 1 to 381 for proSP-B are disclosed in Johansson 1992, FEBS Lett. 301:165-167, Jacobs 1987, J Biol Chem 262(20): 9808-11 and Jacobs 1988, J Biol Chem 263(2): 1093, or Pilot-Matias 1989, DNA 8:75-86 and are deposited in UniProtKB/Swiss-Prot data base under accession numbers P07988; Q96R04 or Genbank accession number P07988.3. The term also encompasses variants of the aforementioned specific SP-B peptides. Such variants have at least the same essential biological and immunological properties as the specific SP-B peptides. In particular, they share the same essential biological and immunological properties if they are detectable by the same specific assays referred to in this specification, e.g., by ELISA assays using polyclonal or monoclonal antibodies specifically recognizing the said SP-B peptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific SP-B peptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The comparison window, preferably, is the entire length of the query sequence or at least 50% of its length. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific SP-B peptides or the aforementioned types of variants as long as these fragments have the essential immunological and biological properties as referred to above. Such fragments may be, e.g., degradation products of the SP-B peptides. Further included are variants which differ due to posttranslational modifications such as phosphorylation or myristylation. Moreover, the aforementioned SP-B peptides may be present as a monomer and/or in dimerized form.

Determining the amount of a SP-B peptide or any other peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) preferably, removing non-bound ligand and other components which may be present in the sample, (c) measuring the amount of bound ligand, i.e. the complex of the peptide and the ligand formed in step (a). The bound ligand, i.e. the ligand or the ligand/peptide complex, will generate an intensity signal which reflects the amount of peptide or polypeptide originally present in the sample. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to, i.e. cross-react with, another peptide, polypeptide or substance present in the sample to be analysed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative.

Binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidinbiotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemiluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

Further suitable techniques for the determination of a polypeptide or peptide are described in the following.

The amount of a peptide or polypeptide may be, also preferably, determined as follows: (a) contacting a solid support comprising a ligand for the peptide or polypeptide as specified above with a sample comprising the peptide or polypeptide, (b) preferably, removing unbound peptide or polypeptide as well as remaining sample material and (c) measuring the amount peptide or polypeptide which is bound to the support. Preferably, the amount of the complex of the ligand and the peptide or polypeptide formed on the solid support is measured. It will be understood that the amount of the complex formed during the determination shall represent the amount of the peptide or polypeptide originally present in the sample. The ligand is, preferably chosen from the group consisting of nucleic acids, peptides, polypeptides, antibodies and aptamers and is, preferably, present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analysed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a subject exhibiting a symptom of an acute cardiovascular event suffers from pulmonary complication, or not. Therefore, the reference amount is to be chosen so that either a difference or a similarity in the compared amounts allows identifying those test subjects which belong into the group of subjects exhibiting a symptom of an acute cardiovascular event and suffering from pulmonary complication.

Accordingly, the term "reference amount" as used herein refers to an amount which allows assessing whether a subject suffering from acute inflammation has an increased risk for mortality. Accordingly, the reference may, e.g., be derived from (i) a subject or group of subjects exhibiting a symptom of an acute cardiovascular event and suffering from pulmonary complication, (ii) a subject or group of subjects exhibiting a symptom of an acute cardiovascular event and not suffering from pulmonary complication or (iii) a clinically apparently healthy subject or a group of such subjects. The reference amount may be used to define and establish a threshold amount. The threshold amount, preferably, allows for a rule-in and/or a rule-out diagnosis. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analysed together, i.e. simultaneously or subsequently, with the test sample.

In one embodiment of the method of the present invention, said reference amount is derived from a subject or a group of subjects exhibiting a symptom of an acute cardiovascular event and known to suffer from a pulmonary complication and wherein an essentially identical or increased amount of the SP-B peptide is indicative for a subject suffering from the said pulmonary complication, whereas a decreased amount of the SP-B peptide is indicative for a subject not suffering from the said pulmonary complication.

In yet another embodiment of the method of the present invention, said reference amount is derived from a subject or a group of subjects exhibiting a symptom of an acute cardiovascular event and known not to suffer from a pulmonary complication and wherein an essentially identical or decreased amount of the SP-B peptide is indicative for a subject not suffering from the said pulmonary complication, whereas an increased amount of the SP-B peptide is indicative for a subject suffering from the said pulmonary complication.

Reference amounts can be calculated for a cohort of subjects (i.e. (i) a subject or group of subjects exhibiting a symptom of an acute cardiovascular event and suffering from pulmonary complication, (ii) a subject or group of subjects exhibiting a symptom of an acute cardiovascular event and not suffering from pulmonary complication or (iii) a clinically apparently healthy subject or a group of such subjects) based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from a pulmonary complication, or not, can be generated, preferably, by establishing a ROC for said cohort as described above and deriving a threshold amount therefrom.

Preferably, the median values for a biomarker determined in a reference population as specified above may be also used as a basis for establishing thresholds. More preferably, the median for proSP-B of about 127 ng/ml found in subjects suffering from decompensated heart failure was found to be a threshold for differentiating between subjects exhibiting a symptom of an acute cardiovascular event either suffering from a pulmonary complication, or not. Also preferably, the value for the 75^{th} percentile value for proSP-B found in this group, i.e. about 211 ng/ml, may be used as well. More preferably, the median for C-terminal fragment of proSP-B of about 296 ng/ml found in subjects suffering from decompensated heart failure was found to be a threshold for differentiating between subjects exhibiting a symptom of an acute cardiovascular event either suffering from a pulmonary complication, or not. Also preferably, the value for the 75^{th} percentile value for the C-terminal fragment of proSP-B found in this group, i.e. about 483 ng/ml, may be used as well. Moreover, further preferred thresholds may, dependent on a desired specificity and sensitivity be derived from the 25^{th} percentiles, medians and/or 75^{th} percentiles recited in the tertiles of the accompanying Examples, below.

More specifically, the following ranges were found in accordance with the present invention to be useful for diagnosing pulmonary congestion: Below the thresholds of about 29 ng/ml for proSP-B or about 60 ng/ml for C-terminal fragment of proSP-B pulmonary congestion can be ruled out. Above these thresholds, pulmonary congestion may be present at a subclinical stage, i.e. no rule out for pulmonary congestion can be made. Below the thresholds of about 127 ng/ml for proSP-B or about 296 ng/ml for C-terminal fragment of proSP-B pulmonary congestion may be present at a subclinical stage, i.e. no rule out can be made. Above these thresholds, pulmonary congestion shall be present at a subclinical or clinical stage. Below the thresholds of about 211 ng/ml for proSP-B or about 483 ng/ml for C-terminal fragment of proSP-B pulmonary congestion shall be present at a subclinical stage. Above these thresholds, pulmonary congestion shall be present at a clinical or clinical stage.

"About" as used in accordance with the present invention means +/- 20%, +/- 10%, +/- 5%, +-2 % or +-/ 1% from the said value.

The method of the present invention may also include a step of establishing a diagnosis or an aid therefor whether or not a subject suffers from a pulmonary complication, or not, based on the result of the comparison described above.

Moreover, the method of the present invention may include further steps of determining at least one biomarker selected from the group consisting of: a natriuretic peptide and, preferably, NT-proBNP, a cardiac troponin, preferably troponin T or I, GDF-15 and soluble Flt-1. As indicated before, increased amounts for these biomarkers are indicative for ischemia (sFlt-1), a functional impairment of the heart (natriuretic peptides or GDF-15) or heart damage (troponin I or T).

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms described elsewhere herein. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin. The degree of identity between two amino acid sequences, in principle, can be determined by algorithms described elsewhere herein. Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

The term "sFlt-1" as used herein refers to polypeptide which is a soluble form of the fms-like tyrosine kinase 1. It was identified in conditioned culture medium of human umbilical vein endothelial cells. The endogenous sFlt1 receptor is chromatographically and immunologically similar to recombinant human sFlt1 and binds [1251] VEGF with a comparable high affinity. Human sFlt1 is shown to form a VEGF-stabilized complex with the extracellular domain of KDR/Flk-1 in vitro. Preferably, sFlt1 refers to human sFlt1 as describe in Kendall 1996, Biochem Biophs Res Commun 226(2): 324-328; for amino acid sequences, see, e.g., also Genebank accession numbers P17948, GI: 125361 for human and BAA24499.1, GI: 2809071 for mouse sFlt-1 (Genebank is available from the NCBI, USA under www.ncbi.nlm.nih.gov/entrez). The term also encompasses variants of the aforementioned human sFlt-1 polypeptides. Such variants have at least the same essential biological and immunological properties as the aforementioned sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt-1, respectively. The degree of identity between two amino acid sequences can be determined by algorithms described elsewhere herein. Variants referred to above may be allelic variants or any other species specific homologs, paralogs, or orthologs. Variants referred to above may be allelic variants or any other species specific homologs, paralogs, or orthologs. Moreover, the variants referred to herein include fragments or subunits of the specific sFlt-1 polypeptides or the aforementioned types of variants as long as these fragments have the essential immunological and biological properties as referred to above. Such fragments may be, e.g., degradation products of the sFlt-1 polypeptides. Variants are deemed to share the same essential biological and immunological properties if they are detectable by the same specific assays referred to in this specification, e.g., by ELISA assays using polyclonal or monoclonal antibodies specifically recognizing the said sFlt-1 polypeptides. A preferred assay is described in the accompanying Examples. Further included are variants which differ due to posttranslational modifications such as phosphorylation or myristylation. sFlt-1 may be detected in bound or free form or as total sFlt-1 amount in a sample.

The term "Growth and Differentiation Factor-15" or "GDF-15" relates to a polypeptide being a member of the transforming growth factor beta (TGFβ) cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated genre-1, and prostate-derived factor (Hromas 1997, Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a ~28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms described elsewhere herein. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

Advantageously, it has been found in the studies underlying the present invention that SP-B peptides, such as proSP-B or the C-terminal fragment of proSP-B, in a body fluid such as blood, plasma or serum can serve as a biomarker that allows for identifying a pulmonary complication in subjects exhibiting a symptom of an acute cardiovascular event. Thanks to the present invention, it is possible to identify those patients with pulmonary complications. The conventional diagnostic approaches of acute cardiovascular events including STEMI or ACS do not adequately address pulmonary congestion or other pulmonary complications. However, the reliable and efficient diagnosis of pulmonary complications allows for an individual therapeutic interventions and/or adaptations. Specifically, fluid or diuretic therapies may be adapted if pulmonary complications are diagnosed since these therapies may influence the pulmonary complication adversely.

In an aspect of the invention, a method for establishing an aid for diagnosing whether a subject exhibiting a symptom of an acute cardiovascular event is suffering from a pulmonary complication, or not, is contemplated, said method comprising:
a) determining the amount of a SP-B peptide in a sample of said subject, said determining comprises (i) bringing the sample into contact with a detection agent that specifically binds to the SP-B peptide for a time sufficient to allow for the formation of a complex of the said detection agent and the SP-B peptide from the sample, (ii) measuring the amount of the formed complex, wherein the said amount of the formed complex is proportional to the amount of the SP-B peptide present in the sample, and (iii) transforming the amount of the formed complex into an amount of the SP-B peptide reflecting the amount of the SP-B peptide present in the sample;
b) comparing said amount to a reference; and
c) establishing an aid for diagnosing a pulmonary complication based on the result of the comparison made in step b).

A suitable detection agent may be, in an aspect, an antibody which is specifically binds to a SP-B peptide, i.e. mature SP-B, proSP-B or the C-terminal fragment of SP-B, in a sample of a subject to be investigated by the method of the invention. Another detection agent that can be applied, in an aspect, may be an aptamere which specifically binds to the SP-B peptide in the sample. In yet an aspect the, sample is removed from the complex formed between the detection agent and the SP-B peptide prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the SP-B peptide present in the sample. It will be understood that the specificity and/or sensitivity of the detection agent to be applied defines the degree of proportion of SP-B peptide comprised in the sample which is capable of being specifically bound. Further details on how the determination can be carried out are also found elsewhere herein. The amount of formed complex shall be transformed into an amount of SP-B peptide reflecting the amount indeed present in the sample. Such an amount, in an aspect, may be essentially the amount present in the sample or may be, in another aspect, an amount which is a certain proportion thereof due to the relationship between the formed complex and the amount present in the original sample.

In yet an aspect of the aforementioned method, step a) may be carried out by an analyzing unit, in an aspect, an analyzing unit as defined elsewhere herein.

In an aspect of the method of the invention, the amount determined in step a) is compared to a reference. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for diagnosing is established based on the comparison carried out in step b) by allocating the subject either into a group of subjects suffering from a pulmonary complication with certain likelihood or a group of subj ects not suffering therefrom. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. Thus, the method may establish an aid of diagnosis which may, in an aspect, require further strengthening of the diagnosis by other techniques. In an aspect of the invention, the aid for diagnosing is established automatically, e.g., assisted by a computer system or the like.

In an aspect of the method of the invention, said method further comprises a step of recommending and/or managing the subject according to the result of the aid of diagnosis established in step c). Such a recommendation may, in an aspect, be an adaptation of life style, nutrition and the like aiming to improve the life circumstances, the application of therapeutic measures, e.g., fluid or diuretic therapy, as set forth elsewhere herein in detail, and/or adapting intensiveness of disease monitoring.

In an aspect of the aforementioned method, steps b) and/or c) are carried out by an evaluation unit as set forth elsewhere herein.

It is to be understood that the definitions and explanations of the terms made above and below apply accordingly for all embodiments described in this specification and the accompanying claims.

The present invention also relates to a method for diagnosing whether a subject exhibiting a symptom of an acute cardiovascular event is at risk of developing a pulmonary complication, or not, said method comprising:
a) determining the amount of a surfactant protein-B peptide (SP-B peptide) in a sample of said subject; and
b) comparing said amount to a reference amount whereby it is diagnosed whether the said subject is at risk of developing a pulmonary complication, or not.

The term "diagnosing whether a subject exhibiting a symptom of an acute cardiovascular event is at risk of developing a pulmonary complication" refers to making a prediction of the likelihood according to which a subject will develop a pulmonary complication within a future time window (i.e., a prognostic window) and determining whether said likelihood is statistically significant increased when compared to the prevalence for pulmonary complications in the respective cohort of subjects. As will be understood, such a prediction will not be correct in all cases. However, the term requires that the risk assessment is correctly made for statistically significant number of cases. How such a statistically significant number can be determined is described already elsewhere in detail. Preferably, a prognostic window envisaged in accordance with the invention is a short term window, preferably lasting at least 1 hour, at least 1 day, at least 2 days at least 3 days up to 1 week. In said short term prognostic window the congestion effects are primarily and directly associated with the acute cardiovascular event or which result from associated events such as papillary muscle rupture or an aneurysm of the heart wall. Moreover, the prognostic window, also preferably, may be a long term prognostic window lasting, preferably, at least 3 weeks, at least 1 month, at least 2 months, at least 3 months, at least 6 months, at least 12 months or up to several years. The congestion effects which occur during this long term prognostic window arise from secondary disorders such as improper cardiac remodeling.

Preferably, the reference amount for the aforementioned method is derived from a subject or a group of subjects exhibiting a symptom of an acute cardiovascular event and known to be at risk for developing a pulmonary complication and wherein an essentially identical or increased amount of the SP-B peptide is indicative for a subject being at risk for developing the said pulmonary complication, whereas a decreased amount of the SP-B peptide is indicative for a subject not being at risk for developing the said pulmonary complication.

Preferably, the reference amount for the aforementioned method may also be derived from a subject or a group of subjects exhibiting a symptom of an acute cardiovascular event and known not to be at risk for developing a pulmonary complication and wherein an essentially identical or decreased amount of the SP-B peptide is indicative for a subject not being at risk for developing the said pulmonary complication, whereas an increased amount of the SP-B peptide is indicative for a subject being at risk for developing the said pulmonary complication.

In a preferred embodiment of the aforementioned methods, said methods further comprise recommending a therapy for treating and/or preventing the said pulmonary complication.

The term "therapy for treating and/or preventing the pulmonary complication" refers to a modification of a fluid application or to a modification or change of drug administration, such as administration of diuretics (e.g., change of a loop diuretic to an aldosterone antagonist). Preferably, a preexisting heart failure therapy can be modified based on the result of the method of the present invention in that the administration of a diuretic or aldosterone antagonist is reduced in order to increase fluid presence or to improve efficacy of fluid applications, if necessary.

In another preferred embodiment of the aforementioned methods, said methods further comprises recommending a monitoring intensiveness.

The term "monitoring intensiveness" as used herein refers to the frequency of monitoring (e.g., weak, regular or close monitoring) and to the extent of investigation carried out per monitoring (e.g. application of chest x-ray, or not).

The present invention also relate to the use of a SP-B peptide or detection agents which specifically bind thereto in a sample of a subject exhibiting a symptom of an acute cardiovascular event for diagnosing whether the subject suffers from a pulmonary complication. Moreover, also encompassed is the use of a SP-B peptide or detection agents which specifically bind thereto in a sample of a subject exhibiting a symptom of an acute cardiovascular event for the manufacture of a pharmaceutical or diagnostic composition for diagnosing whether the subject suffers from a pulmonary complication

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

Further encompassed by the invention is the use of a SP-B peptide or detection agents which specifically bind thereto in a sample of a subject exhibiting a symptom of an acute cardiovascular event for diagnosing whether the subject is at risk of developing a pulmonary complication. Moreover, the invention contemplates the use of a SP-B peptide or detection agents which specifically bind thereto in a sample of a subject exhibiting a symptom of an acute cardiovascular event for the manufacture of a pharmaceutical or diagnostic composition for diagnosing whether the subject is at risk of developing a pulmonary complication

The present invention also relates to a device adapted for carrying out a method of the invention comprising
a) an analyzing unit comprising a detection agent which specifically binds to a SP-B peptide, said unit being adapted for determining the amount of the SP-B peptide in a sample of a subject exhibiting a symptom of an acute cardiovascular event; and
b) an evaluation unit for comparing the determined amounts with reference amounts whereby it can be diagnosed whether the said subject suffers from or is at risk of developing a pulmonary complication, said unit comprising a database with reference amount values and a computer-implemented algorithm carrying out the comparison.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. The analysing unit, preferably, comprises said detection agents in immobilized form on a solid support which is to be contacted to the sample comprising the biomarkers the amount of which is to be determined. Moreover, the analysing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subject or group of subjects as defined above in context with the method of the present invention. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the invention pertains to a kit adapted for carrying out a method of the present invention comprising a detection agent for the SP-B peptide, reference standards as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined amount for the SP-B peptide polypeptide representing a reference amount. Such a standard may represent, e.g., the amount of SP-B peptide from a subject or group of subjects exhibiting a symptom of an acute cardiovascular event and suffering from a pulmonary complication or a subject or group of subjects exhibiting a symptom of an acute cardiovascular event and not suffering from a pulmonary complication or a clinically apparently healthy subject or group thereof.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

All references cited in this specification are herewith incorporated by reference with respect to their entire disclosure content and the disclosure content specifically mentioned in this specification.

### EXAMPLES

The following Examples shall merely illustrate the invention. They shall, whatsoever, not be construed as limiting the scope.

### Example 1: Determination of biomarkers in serum samples

The proSP-B assay uses a mouse monoclonal anti-proSP-B (N-terminus) antibody as a capture and a mouse monoclonal anti-proSP-B (C-terminus) antibody as a detection reagent. The assay principle is a sandwich format. The antibody to the N-terminal pro-sequence binds to an epitope comprised in the peptide sequence ranging from amino acid 160 to 169 of proSP-B. The antibody to the C-terminal pro-sequence binds to an epitope comprised in the peptide sequence ranging from amino acid 323 to 334 of proSP-B. Detection is based on an electrochemiluminescence immunoassay (ECLIA), using a Tris(bipyridyl)-ruthenium(II) complex as label. For carrying out the assay, the biotinylated capture antibody (80 µl), the ruthenium-labeled detection antibody (80 µl), and sample or standard material (10 µl) are incubated in homogeneous phase for 9 min at 37°C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for proSP-B is given in ng/ml.

The C-fragment proSP-B assay uses a first mouse monoclonal anti-proSP-B (C-terminus) antibody as a capture and a second mouse monoclonal anti proSP-B (C-terminus) antibody as a detection reagent. The assay principle is a sandwich format. The antibody to the first C-terminal pro-sequence binds to an epitope comprised in the peptide sequence ranging from amino acid 323 to 334 of proSP-B. The antibody to the second C-terminal pro-sequence binds to an epitope comprised in the peptide sequence ranging from amino acid 285 to 294 of proSP-B. Detection is based on an electrochemiluminescence immunoassay (ECLIA), using a Tris(bipyridyl)-ruthenium(II) complex as label. For carrying out the assay, the biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for C-terminal proSP-B is given in ng/ml.

NT-pro BNP and sensitive Troponin T were determined using commercially available ELECSYS tests.

NT-proBNP was determined with sandwich immunoassays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35,000 pg/ml can be measured.

Troponin T (hsTNT) was also tested using Roche analysers, the test follows the same test principles as described for NT-pro BNP. The high sensitivity Troponin T test used in this study has a sensitivity of 2 pg/ml and can be used on ELECSYS 2010 as well as on Cobas e411 an cobas e601 analysers.

GDF-15 and sFlt1 were determined with sandwich immunoassays using analyzers from Roche/Hitachi, Elecsys or COBAS e-series. Each assay comprises two monoclonal antibodies specific for the respective polypeptide. The first of these antibodies is biotinylated and the second one is labelled with a Tris(2,2'-bipyridyl)ruthenium(II)-complex. In a first incubation step both antibodies are incubated with the sample. A sandwich complex comprising the peptide to be determined and the two different antibodies is formed. In a next incubation step streptavidin-coated beads are added to this complex. The beads bind to the sandwich complexes. The reaction mixture is then aspirated into a measuring cell where the beads are magnetically captured on the surface of an electrode. The application of a voltage then induces a chemiluminescent emission from the ruthenium complex which is measured by a photomultiplier. The emitted amount of light is dependent on the amount of sandwich complexes on the electrode. The measuring range of GDF-15 it was 300 pg/ml to 20,000 pg/ml. For sFlt1, amounts between 10 to 85,000 pg/ml could be measured.

### Example 2: proSP-B and C-terminal fragment of proSP-B are indicators for pulmonary congestion in chest pain patients

Control patients with asymptomatic coronary artery disease (n = 74) and patients with decompensated heart failure were included into the study (n = 92). The control patients with asymptomatic coronary artery disease were clinically stable, had normal kidney function and did not suffer from primary or secondary lung disorders. Moreover, patients with chest pain with and without detectable evidence of cardiac ischemia were included into the study (n = 94). A further group of patients (n = 22) consisted of patients diagnosed with ST elevation myocardial infarction.

The results of the determination of serum levels of several biomarkers are summarized in the following tables.

**Table 1: Results of the control population**

| | NT-proBNP | proSP-B | C-fragment proSP-B |
|---|---|---|---|
| | pg/ml | ng/ml | ng/ml |
| | | | |
| Stable CAD | 144 | 29 | 60 |
| N=74 | 67 - 283 | 23 - 52 | 42 - 104 |
| | | | |
| Decompensated HF | 4478 | 127 | 296 |
| N=92 | 1878 - 8908 | 79 - 211 | 157 - 483 |

Median and 25th and 75th percentiles are given

In the control group patients with CAD who were asymptomatic showed no signs of cardiac decompensation and specifically no evidence of pulmonary congestions as evidenced by rales. In contrast in patients with decompensated heart failure, pulmonary congestion was found clinically to be associated with proSP-B values above 127 ng/ml and even more frequent with proSP-B values above 211 ng/ml, respective concentrations for C-fragment proSP-B were 296 and 483 ng/ml.

A total of 94 patients presenting within two hours with the symptoms of chest pain in the emergency room were included into the study. They were separated into two groups, one group (n = 76) 9 had no detectable ischemia as evidenced by no increased sFlt-1 and a second group had evidence of ischemia at presentation (increased sFlt-1 levels). Follow up samples were also available 6 h after presentation.

**Table 2: Results of the chest pain patients**

| Group 1 | | |
|---|---|---|
| | TP 0 | TP6h |
| proSP-B ng/ml | 55,1 (31,8/105,6) | 52,9 (32/98,4) |
| C-fragment proSP-B ng/ml | 123,8 (79/263) | 127,3 (82/252) |
| NT-proBNP pg/ml | 227 (68/1060) | 320 (92/1220) |
| sensTroponin T pg/ml | 9,2 (3,5/19,3) | 10,2 (3,6/25.3 |
| sFlt-1 pg/ml | 78 (67/89) | 78 (68/95) |
| GDF-15 pg/ml | 1007 (633/1272) | 1091 (868/1576) |

| Group 2 | | |
|---|---|---|
| proSP-B ng/ml | 72,2 (26/ 195) | 90,2 (41/157) |
| C-fragment proSP-B ng/ml | 159 (70/457) | 183 (99 /391) |
| NT-proBNP pg/ml | 373 (70/2902) | 641 (119/3407) |
| sensTroponin T pg/ml | 13 (5/28) | 11,7 (4/33) |
| sFlt-1 pg/ml | 2585 (814/3530) | 75 (67/133) |
| GDF-15 pg/ml | 1237 (814/3530) | 1228 (957/4146) |

When comparing group 1 and group 2 it was evident that non ischemic chest pain patients had less increase in NT-proBNP than ischemic patients and there was no increase in surfactant proteins or troponin T in the non ischemic group, there was an increase in surfactant proteins however in the ischemic group. In general, the ischemic group had more advanced cardiac disease than the non-ischemic group indicated by baseline NT-proBNP, sensitive troponin T and GDF-15 levels.

Evidence of present pulmonary congestion or development of such a condition with the 6 h observation period was found in the following patients.

**Table 3: Case studies**

| Case | proSP-B | C-fragment proSP-B | NT-proBNP | sensTroponin T | sFlt-1 |
|---|---|---|---|---|---|
| | ng/ml | ng/ml | pg/ml | pg/ml | pg/ml |
| **5** | **139** | **332** | **94** | **3** | **59** |
| 8 | 242 | 1755 | 94 | 7 | 2101 |
| **9** | **127** | **260** | **29** | **3** | **2267** |
| **10** | **218** | **521** | **248** | **53** | **68** |
| **13** | **137** | **371** | **5846** | **27** | **116** |
| 15 | 165 | 561 | 86 | 67 | 73 |
| 22 | 218 | 407 | 2139 | 407 | 74 |
| **23** | **163** | **527** | **332** | **7** | **65** |
| 24 | 288 | 771 | 18827 | 48 | 4151 |
| **33** | **158** | **400** | **3400** | **18** | **1664** |
| 35 | 233 | 483 | 2763 | 45 | 2829 |
| **37** | **157** | **402** | **4318** | **1258** | **2533** |
| **44** | **140** | **432** | **6142** | **28** | **3207** |
| **47** | **148** | **338** | **989** | **23** | **87** |
| 50 | 295 | 713 | 1292 | 17 | 4855 |
| 60 | 796 | 1598 | 3043 | 22 | 3610 |
| 70 | 191 | 505 | 5851 | 142 | 127 |
| **75** | **132** | **225** | **2131** | **33** | **87** |
| 94 | 211 | 521 | 3617 | 108 | 109 |
| 97 | 378 | 1042 | 343 | 4 | 1610 |

| | | | | | |
|---|---|---|---|---|---|
| Note: Cases which developed pulmonary congestion are marked in **bold,** the other cases exhibited already pulmonary congestion. | | | | | |

As can be seen among the individual cases, evidence of pulmonary congestion was associated with underlying heart failure in a subset of cases or previous necrosis without detectable ischemia in some cases or acute ischemia in half of the cases or a combination of different factors. As is also evident none of the reference tests (NT-proBNP, sensTroponin T or sFlt-1 can replace the information provided by proSP-B. Of note proSP-B remained largely unchanged during the 6 h observation period.

In addition a total of 22 patients with ST elevation myocardial infarction was included into the study. This is shown in the Table below: Results are given at presentation and at 24 h follow up.

**Table 4: Time course study of STEMI patients**

| | **T 0** | **T 24** |
|---|---|---|
| proSP-B ng/ml | 74 (56/97) | 89 (66/152) |
| C-fragment proSP-B ng/ml | 154 (120/285) | 256 (131/321) |
| NT-proBNP pg/ml | 683 (148(2631) | 3131 (2284/5151) |
| sens Troponin T pg/ml | 219 (57/633) | 1343 (944/ 5016) |
| sFlt-1 pg/ml | 766 (311/1271) | 127 (111/182) |

As in patients with chest pain pulmonary congestion was associated with baseline cardiac function. Patients with STEMI who had initially an unimpaired cardiac function did not develop pulmonary congestion or increase of pro SP-B although their NT-pro BNP and troponin T levels increased significantly This is shown in specific Examples below (cases 1 and 12). In contrast, patients with impaired cardiac function showed pulmonary congestion and in few cases also an increase of proSP-B (Cases 32 and 36)

**Table 5: Case studies**

| Case | proSP-B | C-fragment proSP-B | NT-proBNP | troponin T |
|---|---|---|---|---|
| | ng/ml | ng/ml | pg/ml | pg/ml |
| 1, T 0 | 73, | 154 | 77 | 174 |
| T 24 | 85, | 160 | 869 | 2635 |
| 12, T0 | 71 | 133 | 61 | 18 |
| T 24 | 63 | 117 | 860 | 1057 |
| 32 T 0 | 144 | 286 | 1587 | 193 |
| T 24 | 151 | 303 | 5016 | 1084 |
| 36 T 0 | 168 | 415 | 10109 | 4331 |
| T 24 | 251 | 513 | 16636 | 13270 |

A further time course of the biomarkers was measured in order to determine the robustness of the proSP-B and C-terminal fragment proSP-B levels over time. Specifically, a total of 22 patients with clinically stable heart failure were included into the study, all patients were on standard heart failure therapy including beta blockers, ACE inhibitors and diuretics. Patients were seen at 2 weeks interval. During that period treatment was not changed. Moreover they have no change in symptoms und their weight did not differ of more than 2 kg bodyweight 4 weeks before study start and during the study. All patients had normal kidney function. Data are summarizes in the Table 6, below.

**Table 6: Time course measurements**

| | NT-pro BNP | pro-SP-B | c frag pro-SP-B | TroponinT |
|---|---|---|---|---|
| | pg/ml | ng/ml | ng/ml | pg/ml |
| TP 1 | 367 (216-924) | 57 (33-80) | 119 (69-162) | 8 (4-15) |
| TP 2 | 362 (229-971) | 52 (31-75) | 111 (66-156) | 7 (3-12) |
| TP 3 | 374 (237-973) | 52 (29-75) | 109 (61-154) | 7 (4-12) |

As can be seen from the table pro-SP-B and c fragment pro SP-B are very stable and show little variation in this population indicating than minor changes in pro SP-B and c fragment pro SP-B respectively reflect changes in pulmonary-alveolar status.

### Conclusion:

Present and future pulmonary congestion was clearly associated with increased levels of pro-SP-B and c fragment proSP-B respectively. Increased concentrations of proSP-B were associated with increased NT-proBNP concentrations (heart failure), ischemia (increased sFlt-1 levels) and/or increased markers of necrosis, however these markers do not capture the information provided by proSP-B.

In two cases involuntarily pulmonary congestion clinically worsened, this was the case after increased fluid application in one patient (case No 13) and reduction in diuretics (case No 22).

The data obtained allow for drawing the following conclusions:

Patients with increased proSP-B levels and acute events deserve a more intense monitoring as compared to those with low or normal proSP-B concentrations because of the possibility of worsening of heart failure.

For the same reason fluid application in patients with increased pro-SP-B levels need to be controlled more carefully, the same is true for the use of diurectis and, specifically, its discontinuation or with respect to drug change (e.g. from rapidly acting loop diuretic to slow acting aldosterone antagonist). Furthermore patients with low proSP-B levels do not require (prophylactic) diuretics even when cardiac dysfunction and/or necrosis increase (and specifically if blood pressure is low).

In summary, proSP-B or C-terminal fragment of proSP-B adds important information to the current information provided by other cardiac markers such as NT-proBNP, troponin T and sFlt-1 in patients with acute cardiovascular events and helps to direct patient surveillance and treatment.

## Claims

1. A method for diagnosing whether a subject exhibiting a symptom of an acute cardiovascular event suffers from a pulmonary complication, or not, said method comprising:
a) determining the amount of a surfactant protein-B peptide (SP-B peptide) in a sample of said subject; and
b) comparing said amount to a reference amount whereby it is diagnosed whether the said subject suffers from a pulmonary complication, or not.

2. The method of claim 1, wherein said reference amount is derived from a subject or a group of subjects exhibiting a symptom of an acute cardiovascular event and known to suffer from a pulmonary complication and wherein an essentially identical or increased amount of the SP-B peptide is indicative for a subject suffering from the said pulmonary complication, whereas a decreased amount of the SP-B peptide is indicative for a subject not suffering from the said pulmonary complication.

3. The method of claim 1, wherein said reference amount is derived from a subject or a group of subjects exhibiting a symptom of an acute cardiovascular event and known not to suffer from a pulmonary complication and wherein an essentially identical or decreased amount of the SP-B peptide is indicative for a subject not suffering from the said pulmonary complication, whereas an increased amount of the SP-B peptide is indicative for a subject suffering from the said pulmonary complication.

4. A method for diagnosing whether a subject exhibiting a symptom of an acute cardiovascular event is at risk of developing a pulmonary complication, or not, said method comprising:
a) determining the amount of a surfactant protein-B peptide (SP-B peptide) in a sample of said subject; and
b) comparing said amount to a reference amount whereby it is diagnosed whether the said subject is at risk of developing a pulmonary complication, or not.

5. The method of claim 4, wherein said reference amount is derived from a subject or a group of subjects exhibiting a symptom of an acute cardiovascular event and known to be at risk for developing a pulmonary complication and wherein an essentially identical or increased amount of the SP-B peptide is indicative for a subject being at risk for developing the said pulmonary complication, whereas a decreased amount of the SP-B peptide is indicative for a subject not being at risk for developing the said pulmonary complication.

6. The method of claim 4, wherein said reference amount is derived from a subject or a group of subjects exhibiting a symptom of an acute cardiovascular event and known not to be at risk for developing a pulmonary complication and wherein an essentially identical or decreased amount of the SP-B peptide is indicative for a subject not being at risk for developing the said pulmonary complication, whereas an increased amount of the SP-B peptide is indicative for a subject being at risk for developing the said pulmonary complication.

7. The method of any one of claims 1 to 6, wherein said pulmonary complication is pulmonary congestion or pulmonary edema.

8. The method of any one of claims 1 to 7, wherein said SP-B peptide is selected from the group consisting of: proSP-B, mature SP-B and C-terminal fragment of pro SP-B.

9. The method of any one of claims 1 to 9, wherein said subject suffers from decompensated heart failure, exhibits at least chest pain or suffers from ST-elevation myocardial infarction (STEMI).

10. The method of any one of claims 1 to 9, wherein said method further comprises recommending a therapy for treating and/or preventing the said pulmonary complication.

11. The method of any one of claims 1 to 10, wherein said method further comprises recommending a monitoring intensiveness.

12. Use of a SP-B peptide or detection agents which specifically bind thereto in a sample of a subject exhibiting a symptom of an acute cardiovascular event for diagnosing whether the subject suffers from a pulmonary complication.

13. Use of a SP-B peptide or detection agents which specifically bind thereto in a sample of a subject exhibiting a symptom of an acute cardiovascular event for diagnosing whether the subject is at risk of developing a pulmonary complication.

14. A device adapted for carrying out the method of any one of claims 1 to 11 comprising
a) an analyzing unit comprising a detection agent which specifically binds to a SP-B peptide, said unit being adapted for determining the amount of the SP-B peptide in a sample of a subject exhibiting a symptom of an acute cardiovascular event; and
b) an evaluation unit for comparing the determined amounts with reference amounts whereby it can be diagnosed whether the said subject suffers from or is at risk of developing a pulmonary complication, said unit comprising a database with reference amount values and a computer-implemented algorithm carrying out the comparison.

15. A kit adapted for carrying out the method of any one of claims 1 to 11 comprising a detection agent for the SP-B peptide, reference standards as well as instructions for carrying out the said method.
